# EUROPEAN PATENT SPECIFICATION

(11) **EP 4 125 806 B1**
(45) Date of publication and mention of the grant of the patent: **16.10.2024**
(21) Application number: 21715892.2
(22) Date of filing: 31.03.2021
(51) Int. Cl.: A61K 9/00, A61K 38/28, A61K 9/48

(54) **A FLEXIBLE FOIL FOR THE DELIVERY OF THERAPEUTIC CARGOS**
FLEXIBLE FOLIE ZUR ABGABE VON THERAPEUTISCHEN LADUNGEN
FEUILLE FLEXIBLE POUR LA LIVRAISON DE CARGAISONS THÉRAPEUTIQUES

(30) Priority: 31.03.2020 EP 20167006
(43) Date of publication of application: 08.02.2023
(73) Proprietor: Danmarks Tekniske Universitet, 2800 Kongens Lyngby (DK); University of Copenhagen, 1165 Copenhagen K (DK)
(72) Inventor: BOISEN, Anja, 2800 Kongens Lyngby (DK); THAMDRUP, Lasse Højlund Eklund, 2800 Kongens Lyngby (DK); JØRGENSEN, Jacob Rune, 1367 Copenhagen K (DK); RADES, Thomas, 2200 Copenhagen N (DK); MÜLLERTZ, Anette, 2920 Charlottenlund (DK); GUYAN, Khorshid Kam, 2800 Kongens Lyngby (DK); GHAVAMI, Mahdi, 2800 Kongens Lyngby (DK)
(74) Representative: Høiberg P/S
(86) International application number: PCT/EP2021/058473
(87) International publication number: WO 2021/198353

(56) References cited:
- EP-A1- 3 461 478
- WO-A1-2014/159604
- DE-A1- 19 849 848
- US-A1- 2014 135 698
- US-A1- 2014 200 604

## Description

### Technical field

The present invention relates to a drug delivery platform. In particular, it relates to a foil-based drug delivery platform for delivery to the intestine.

### Summary

The present inventors have invented a novel drug delivery platform that facilitates drug delivery through the intestinal wall and seeding of probiotics in the intestinal mucus. The drug delivery platform mediates uptake of drugs and other therapeutically active compositions by creating a high, local concentration of the drug, the therapeutically active compound, or the probiotic composition in close proximity to the intestinal wall. The drug delivery platform ensures efficient absorption of its cargo, which allows for lower drug dosages to be employed. It is fitted with microstructures which can improve drug absorption. The microstructures facilitate interaction with, deformation of, and/or penetration of the mucus layer on the intestinal wall and thereby facilitate absorption through the intestinal wall. Due to the close proximity to the intestinal wall, the therapeutically active compound is protected against the luminal digestive enzymes and microorganisms capable of degrading such compounds before they are absorbed through the intestinal wall. The platform can be further loaded or coated with excipients to tailor the release profile of the loaded pharmaceutical compositions.

The inventors of the present disclosure have produced a rolled foil comprising on its convex surface a pharmaceutical composition. Upon administration to the intestine, the foil unrolls and establishes contact to the interior mucus lining of the intestinal wall. The pharmaceutical composition is thus brought into close proximity of the interior mucus lining the intestinal wall, which provides for surprisingly improved uptake of the pharmaceutical composition through the intestinal wall to the blood circulation.

A first aspect of the present disclosure provides for a rolled foil having on the convex surface of the rolled foil a pharmaceutical composition, wherein the rolled foil and the pharmaceutical composition are comprised inside a covering layer, wherein the convex surface of the rolled foil exerts a radial pressure when unrolled to a diameter of a designated intestine, wherein the convex surface comprises microstructures, and wherein the length of the rolled foil is at least half the length of the inner circumference of the designated intestine.

A second aspect of the disclosure provides for a method of manufacturing the rolled foil of the first aspect comprising the steps of:
a. providing a foil that exerts a radial pressure when rolled to a diameter of the designated intestine wherein the foil has a length of at least half the circumference of the designated intestine,
b. loading the foil with the pharmaceutical composition, and optionally applying a sealing layer,
c. rolling the foil, and
d. covering the rolled foil in a covering layer.

The invention is defined by the claims. Any subject matter falling outside the scope of the claims is provided for information purposes only.

DE 198 49 848 A1 discloses a foil for administration of an active agent.

### Description of Drawings

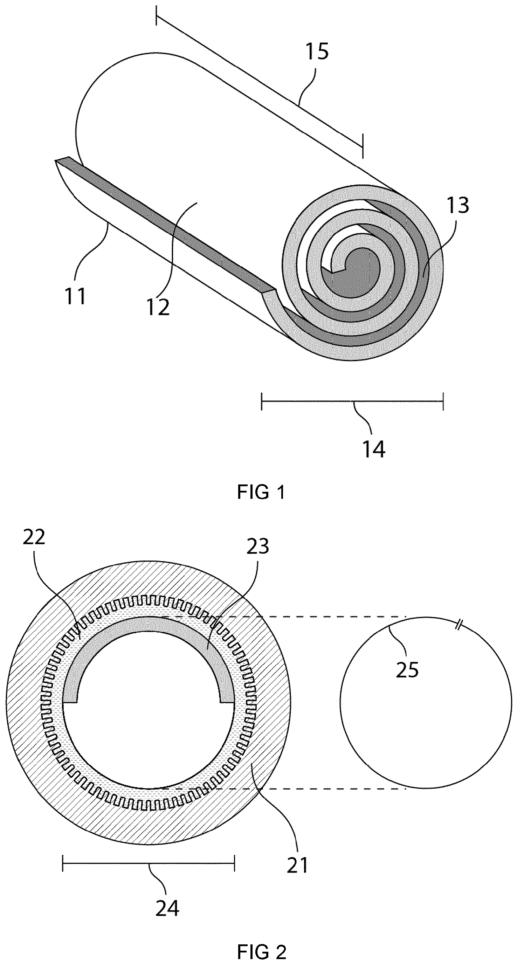

Figure 1: Schematic overview of a rolled foil (11) showing the convex, outer surface (12) and the inner, concave surface (13). Definitions of the outer diameter of the rolled foil (14) and the width of the foil (15) are shown.
Figure 2: Cross sectional view of a designated intestine showing the intestinal wall (21), mucus layer lining the intestinal wall (22), and a foil unrolled to the diameter of the designated intestine (23). Definitions of the inner diameter of the designated intestine (24) and inner circumference of the designated intestine (25) are shown.
Figure 3: Fabrication and preparation steps of drug loaded polydimethylsiloxane (PDMS) foil including scanning electron microscopy (SEM) images. Scale bars: 400 µm.
Figure 4: Cross-sectional view of the rolled PDMS foil inside a size 9 gelatin capsule. Scale bar: 1 mm.
Figure 5: Disintegration of a size 9 gelatin capsule and PDMS foil fully unrolled in water at 37 °C. Specified time in MM:SS. The foil is in a fully unrolled conformation at 1:33.
Figure 6: *in vitro* release of insulin at pH 4 followed by pH 7, mean ± standard deviation (n = 3). Release at pH 4 is negligible over 60 minutes. Upon adjusting the pH to 7, release is complete after 180 minutes.
Figure 7: Coated size 9 gelatin capsule loaded with a magnet (arrow) and a coated PDMS foil loaded with insulin, sodium dodecyl sulfate (SDS) and soybean trypsin inhibitor (STI). Scale bar: 2 mm.
Figure 8: Retrieved foil after the in vivo study placed next to a size 9 gelatin capsule for comparison. Scale bar: 2 mm.
Figure 9: Plasma insulin concentrations in rats shown as mean + standard error of the mean (n = 3-5); full graph and zoom. Plasma levels of insulin show a maximum value of 8 µU/mL after 30 minutes, which is a significant increase over control of SC inj. of saline.

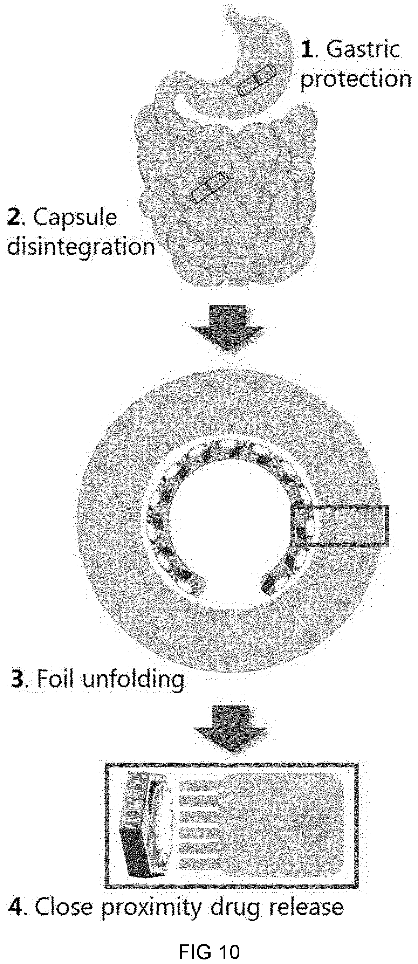

Figure 10: Principle of the delivery system: Intestinal capsule disintegration is followed by foil unrolling and unidirectional release of the pharmaceutical composition in close proximity to the epithelium.

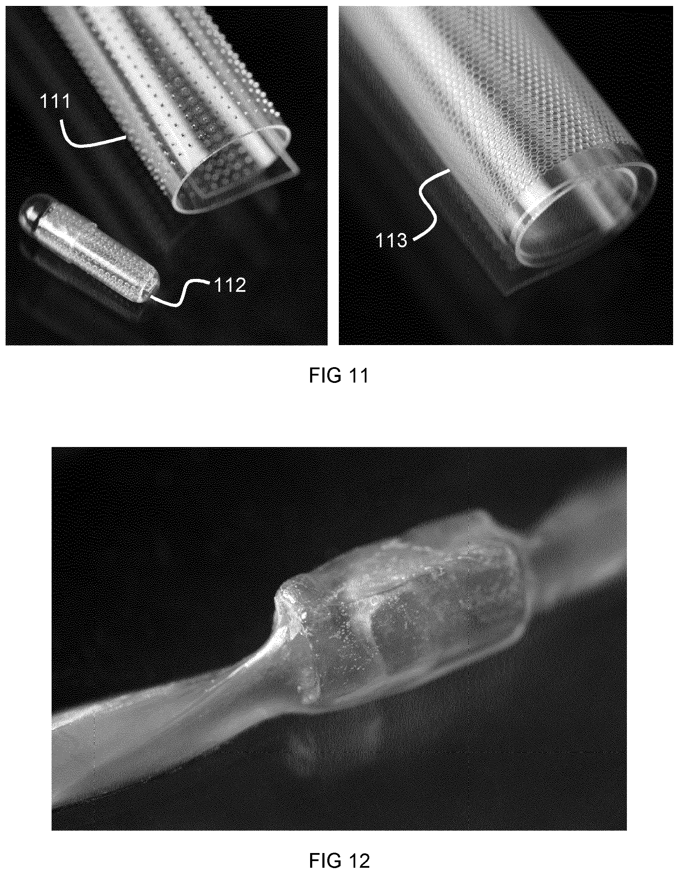

Figure 11: Foils of the present disclosure showing a large sheet of foil having needle-like microstructures on its convex surface (111); a rolled foil having cylindrical compartments defined by walls, comprised within a capsule (112); and a large sheet of foil having hexagonal compartments on its convex surface (113). The large foils may be cut into smaller pieces of desired dimensions.
Figure 12: An unrolled foil comprised within an isolated rat intestine. The unrolled foil shows close contact to the mucus lining of the intestinal wall.

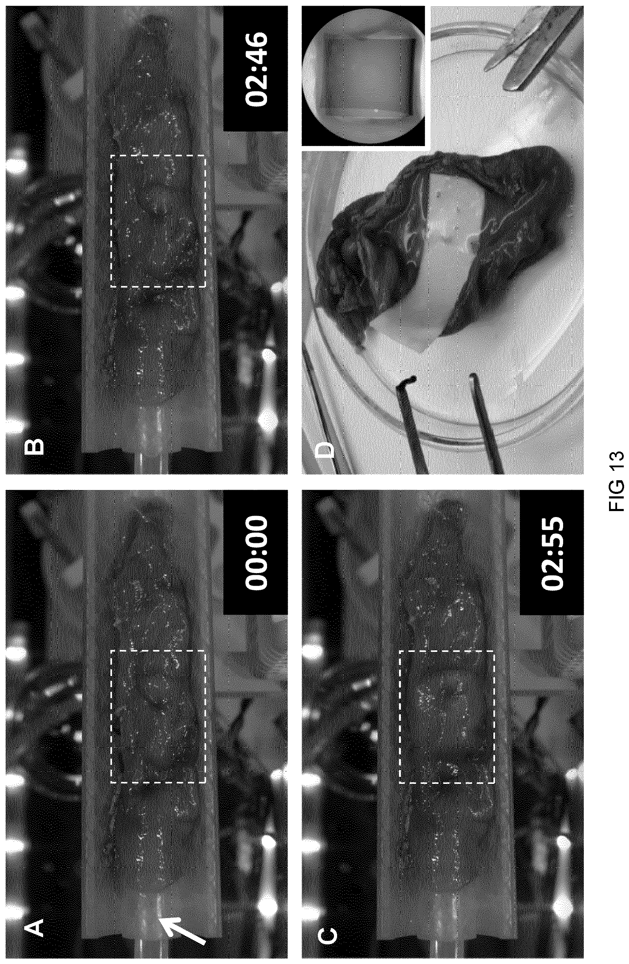

Figure 13: Still pictures from a movie showing the dynamic unrolling of a PDMS foil inside a pig small intestine. The specified time is MM:SS, the region of interest has been marked in all still pictures and the liquid entry point has been marked with a white arrow for t=00:00 (A). The employed PDMS foil has the following dimensions: length=50 mm, width=15 mm and thickness=700 µm. A contrast agent, BaSO₄, was dispersed in the PDMS material and the foil was stored in a size 0 hard shell gelatin capsule. The intestinal segment was perfused with a phosphate buffered saline solution at 37°C. The foil started unrolling at t=02:46 (B) and the steady-state conformation was reached at t=02:55 (C) (i.e. unrolling occurred over approximately 10 s). The foil caused a local expansion of the intestine as it pressed itself against the interior of the tissue, evidencing that the foil exerts a substantial pressure on the wall of the intestine, however not to an extent wherein the foil damages the intestinal tissue.
Figure 13 D shows the dissected intestine with the exposed elastomeric foil, further demonstrating the close contact between the foil and the intestinal wall. As BaSO₄ contrast agent was dispersed in the foil material, the appearance of the foil is opaque. The inset of Figure 13 D is a planar X-ray image of the intact intestine, showing the unrolled conformation of the foil. The unrolled foil exhibited a close contact between its convex surface and the intestinal wall, substantially over the entire surface of the foil.

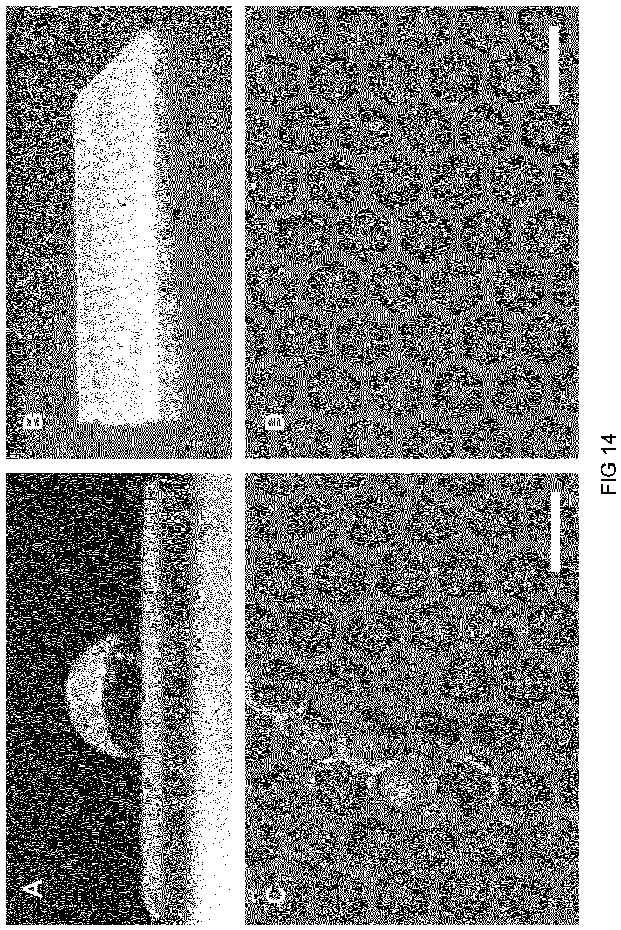

Figure 14: Surface treatment of PDMS foils to improve adhesive properties. A: Native PDMS foil showing a water contact angle (WCA) of approximately 110°. B: A PDMS foil having undergone surface treatment with UV-ozone and polyvinyl alcohol exhibits a significantly reduced WCA, evidencing its increased surface energy. C: A PDMS foil having undergone no surface treatment, spray coated with sealing layer. The sealing layer peels off as the adhesion to the PDMS foil is insufficient. D: A spray coated PDMS foil having undergone surface treatment with UV-generated O₃ and polyvinyl alcohol. The spray coated sealing layer remains intact, even after rolling and unrolling of the PDMS foil. Scale bars correspond to 500 µm.

### Detailed description

### Definitions

In the present disclosure, the term "pharmaceutical composition" refers to a composition intended for conferring some physiological effect to an organism to which the pharmaceutical composition is applied by the action of a therapeutically active compound contained in the composition. Pharmaceutical compositions specifically comprise vaccines. As used herein, the term "pharmaceutical compositions" may also relate to probiotic compositions.

The term "probiotic composition" means a composition comprising a microorganism that exerts beneficial effects on the health of the host.

By "foil" is meant a flexible material in the shape of a sheet. The sheet can be rectangular, elliptic, have the shape of another polygon, optionally with rounded corners.

As used herein, the term "conformation" of a foil is taken to mean the current three-dimensional shape or overall structure of the foil, such as a "rolled foil" or an "unrolled foil". The conformation of a foil determines the amount of stress built up in the bulk foil material. Thus, a foil in a "rolled conformation" possesses a considerable amount of stress in the bulk material that constitutes the foil, which can effect changing of the conformation of the foil, such as unrolling of the foil. Conversely, a foil in a "fully unrolled conformation" or a "non-rolled conformation" will possess a negligible amount of stress in the bulk material, and thus there will be little drive for the foil to change its conformation. Alternatively, the stress built up in a fully unrolled conformation or a non-rolled conformation of the foil is composed of different, counteracting partial stresses such that there will be little overall drive for a change in conformation of the foil.

By "rolled" foil and "fully rolled" foil is meant a foil that is in a rolled conformation possessing some amount of stress.

By "unrolled" is meant a conformation of the foil that is not rolled or fully rolled. Thus, as used herein, expressions such as "when unrolled to a diameter of" specifies a conformation of the foil wherein the foil is partly unrolled such that it forms a round shape or arc-segment shape, and that said shape formed by the foil in said conformation has an average diameter of approximately the specified amount. Alternatively, expressions such as "when unrolled to a circumference of" specifies a conformation of the foil wherein the foil is partly unrolled such that it forms a round shape having a circumference or diameter specified, or such that it forms a arc-segment for which the corresponding round shape has the specified circumference or diameter.

By "fully unrolled" foil is meant a foil possessing an overall neutral amount of stress, such as a negligible amount of stress, counteracting partial stress, or no stress at all. Due to the lack of stress, the foil is in a stable conformation, and thus there is no further drive for the foil to deform.

By "radial pressure" is meant the force exerted by the foil on the intestinal mucus layer per area of contact between the foil and the intestinal mucus layer. The radial pressure acts in a direction away from the convex surface of the foil. The pressure is substantially perpendicular to the convex surface of the foil. In certain embodiments of the disclosure, "pressure" and "radial pressure" are used synonymously.

The term "designated intestine" refers to any part of the intestine to which delivery of pharmaceutical composition is targeted.

As used herein, the term "inner diameter" of a designated intestine relates to the distance across the intestinal luminal space. It is acknowledged that the intestine is not perfectly circular, and that the diameter changes depending on peristalsis and the amount of content presently in the intestine. Values for the average, inner diameter of the intestines are nevertheless known.

As used herein, the term "inner circumference" of a designated intestine relates to the circumference of the intestine as measured at a macroscopic level. It is acknowledged that the inside wall of the intestine comprises structures such as folds, villi, microvilli, which make the circumference large at the microscopic level. However, as used herein, the "inner circumference" relates to the circumference as assessed at the macroscopic level.

As used herein, the term "convex surface" refers to the side of the foil that is situated on the outer surface of the rolled foil. Namely, it is the surface of the foil that has a positive curvature when the foil is rolled. This side of the foil surface will engage with the intestine wall when the foil is unrolled. However, the term is also used to refer to the surface of the foil that becomes convex once the foil is rolled, even if that surface may not possess any curvature during production of the foils or after the foils unroll. When the foil is in the rolled conformation, the stress of the convex surface is tensile. The terms "convex surface", "convex outer surface", "outer convex surface" and "outer surface" in relation to the foil are used interchangeably herein.

As used herein, the term "concave surface" refers to the side of the foil that is situated on the inside of the rolled foil. Namely, it is the surface of the foil that has a negative curvature when the foil is rolled. However, the term is also used to refer to the surface of the foil that becomes concave once the foil is rolled, even if that surface may not possess any curvature during production of the foils or after the foils unroll. When the foil is in the rolled conformation, the stress of the concave surface is compressive. The terms "concave surface", "concave inner surface", "inner concave surface" and "inner surface" in relation to the foil are used interchangeably herein.

As used herein, the phrase "having on the convex surface" is taken to mean both compositions deposited on the convex surface of the foils, but also compositions comprised within the foil material.

### Rolled foils

The present disclosure relates to a rolled foil for delivery of pharmaceutical compositions to the intestine. The rolled foil comprises an outer, convex surface, which further comprises the pharmaceutical composition and microstructures. Upon administration to a designated intestine, the rolled foil unrolls and establishes contact between its outer, convex surface and the mucus lining the intestinal wall. The pharmaceutical composition is released from the foil in the restricted space between the foil and the intestinal wall. This effects a higher concentration of the pharmaceutical composition in the local environment near the intestinal wall than in the rest of the intestinal lumen.

In a preferred embodiment, the foil is administered orally. Thus, in order to prevent that the foil unrolls before reaching the designated intestine, the rolled foil is covered in a covering layer, which is capable of retaining the foil in a rolled conformation. This covering layer dissolves upon arriving in the designated intestine, releasing the rolled foil. Upon release from the covering layer in the designated intestine, the rolled foil unrolls and establishes contact between its convex surface and the intestinal wall. Accordingly, one embodiment of the present disclosure provides for a rolled foil having on the convex surface a pharmaceutical composition, wherein the rolled foil having on the convex surface a pharmaceutical composition is comprised inside a covering layer.

The internal stress of the foil is not fully relaxed when the foil assumes a conformation wherein it is unrolled to a diameter of a designated intestine, i.e., the foil has an intrinsic drive to further unroll. Because the foil is located within the confined space of the designated intestine, further unrolling is restricted and governed by the mechanical properties of the designated intestine. The unrelaxed stress results in a radial pressure exerted at the interface between the convex surface of the foil and the interior surface of the designated intestine. In one embodiment of the disclosure, this contact pressure will assume its maximum values at the extremities of the foil, i.e. near the edges running along the width of the foil. In addition to the pressure exerted by the foil on the intestine, peristalsis effects a fluctuating and opposite pressure on the foil. The conformation of the foil and the radial pressure exerted by the foil on the mucus lining the designated intestine thus fluctuates in response to the peristalsis. During these fluctuations, the convex surface of the foil retains its contact to the mucus lining the designated intestine. Thus, in a preferred embodiment, the unrolled foil exerts a pressure on the mucus lining the intestine wall, such as a radial pressure, to ensure close contact between the convex surface of the foil and the mucus lining the intestine.

It is important that the maximum pressure associated with the conformation of the unrolled foil does not inflict damage on the intestinal tissue. Said tissue is rather flexible having a destructive strain of 140 % at a maximum stress of 0.9 MPa (Egorov, V. I., Schastlivtsev, I. V., Prut, E. V., Baranov, A. O. & Turusov, R. A. Mechanical properties of the human gastrointestinal tract. J. Biomech. 35, 1417-1425 (2002)). Thus, one embodiment of the present disclosure provides for a rolled foil having on the convex surface a pharmaceutical composition, wherein the rolled foil having on the convex surface a pharmaceutical composition is comprised inside a covering layer, wherein the rolled foil exerts a maximum radial pressure of less than 1000 kPa, such as less than 900 kPa, when unrolled to a diameter of a designated intestine. One embodiment of the present disclosure provides for a rolled foil having on the convex surface a pharmaceutical composition, wherein the rolled foil having on the convex surface a pharmaceutical composition is comprised inside a covering layer, wherein the pressure exerted by the rolled foil does not result in tissue deformations approaching 140 % when unrolled to a diameter of a designated intestine.

The pressure exerted by the intestine during peristalsis amounts to a few kPa, such as 2-3 kPa (Scott, S. M. et al. The nocturnal jejunal migrating motor complex: Defining normal ranges by study of 51 healthy adult volunteers and meta-analysis. Neurogastroenterol. Motil. 18, 927-935 (2006)). In one embodiment of the present disclosure, the rolled foil exerts a radial pressure on the mucus lining the intestinal wall which is lower than 2-3 kPa. In this case, the forces exerted on the foil by the intestine during peristalsis are able to deform the foil. As is evident from the examples disclosed herein, the foil of the present disclosure exerts a pressure on the intestinal wall which is lower than 2-3 kPa. In one embodiment of the present disclosure, the foil of the disclosure exerts a pressure on the intestinal wall which is 0.1 to 3000 Pa. In one embodiment of the present disclosure, the foil exerts a pressure on the intestinal wall which is 0.1 to 1 Pa, such as 1 to 5 Pa, such as 5 to 10 Pa, such as 10 to 20 Pa, such as 20 to 50 Pa, such as 50 to 200 Pa, such as 200 to 500 Pa, such as 500 to 1000 Pa, such as 1000 to 2000 Pa, such as 2000 to 3000 Pa. In another embodiment of the present disclosure, the rolled foil exerts a radial pressure on the mucus lining the intestinal wall which is higher than 2-3 kPa. In this case, the forces exerted on the foil by the intestine during peristalsis is not able to deform the foil considerably. In one embodiment of the disclosure, the foil exerts a pressure on the intestinal wall which is 3000 to 5000 Pa, such as 5000 to 7000 Pa, such as 7000 to 10000 Pa, such as 10 to 50 kPa, such as 50 to 100 kPa.

The present disclosure relates to a foil that releases a pharmaceutical composition in proximity of the intestinal wall. Because the pharmaceutical composition is released at the interface between the convex surface of the foil and the intestinal wall, it is preferred that the convex surface of the foil establishes contact over a substantial area of the convex surface of the foil. This is achieved with foils of the present disclosure because the length of the foils is at least half the circumference of the designated intestine. Such foils will, upon unrolling in the designated intestine, line the intestinal wall ensuring a substantial contact between the convex surface of the foil and the intestinal wall. Accordingly, one embodiment of the present disclosure provides for a rolled foil having on the convex surface a pharmaceutical composition, wherein the rolled foil having on the convex surface a pharmaceutical composition is comprised inside a covering layer, and wherein the length of the rolled foil is at least half the length of the inner circumference of the designated intestine. For a foil having a length of more than the inner circumference of the designated intestine, such as 1.5 times the length of the inner circumference, the ends of the foil may overlap as the foil unrolls to the diameter of the designated intestine. Such overlap is allowable as it may account for variations in the diameters of the designated intestine between individuals. Thus, in one embodiment of the present disclosure, a foil is provided having a length of 1.5 times the length of the inner circumference of the designated intestine. Should the ends of the foil unrolled to a diameter of a designated intestine overlap, the exposure of one of the ends of the foil to the mucus layer will be minimal, which will limit the uptake of any pharmaceutical composition loaded on this part of the foil. Thus, it is advantageous that the overlap is minimal. This is achieved when the foil length is close to the length of the inner circumference of the designated intestine. Accordingly, in one embodiment of the present disclosure, a foil is provided having a length that corresponds to the inner circumference of the designated intestine.

The length of the foil as used herein is the dimension of the foil that is orthogonal to both the width of the foil and the thickness of the foil. The foils herein are rolled along the length of the foils. The length of the foil is substantially independent of the conformation of the foil.

In one embodiment of the present disclosure, the foil is surface-treated to improve adhesion of the pharmaceutical composition and/or the sealing layer. Insufficient surface adhesion may be due to the surface energy of the foil material. In particular, the foil material may exhibit a low intrinsic surface energy. In this case, the adhesive properties of the foil material can be improved by increasing the surface energy of the foil. In one embodiment, the surface treatment comprises introducing high surface energy moieties onto the foil material. In a specific embodiment of the present disclosure, the surface treatment comprises UV-ozone exposure followed by immersion in polyvinyl alcohol. In one embodiment, the foil is a surface-treated PDMS foil. The surface energy of a material can be assessed by measuring its water contact angle (WCA).

### Dimensions of the foils

The rolled foils of the present disclosure are preferably administered orally. The rolled foils of the present disclosure can be administered orally because they are of a size when rolled, which can be swallowed. To achieve a size of the rolled foil which can be swallowed, the rolled foil is rolled such that it comprises a plurality of turns. Thus, in one embodiment of the present disclosure, a rolled foil is provided wherein the rolled foil comprises one or more turns, such as at least two turns, such as at least three turns. For larger designated intestines, the length of the foil is larger, and thus even more turns may be required to reduce the size of the rolled foil. Thus, in a further embodiment of the disclosure, the rolled foil comprises at least 6 turns, such as 7 or 8 turns.

The rolled foil possesses an outer diameter which facilitates incorporation in a standard size capsule, such as for instance the 000, 00E, or 00 capsules. In particular, such capsules have inner diameters of less than 9.55 mm such as less than 9.40 mm, less than 8.25 mm, or less than 8.00 mm. Thus, in one embodiment of the present disclosure, the rolled foil has an outer diameter less than 9.55 mm such as less than 9.40 mm. In another embodiment of the present disclosure, the rolled foil has an outer diameter less than 8.25 mm. In yet another embodiment, the rolled foil has an outer diameter less than 8.00 mm.

The size of the rolled foil facilitates incorporation in a standard size capsule, such as for instance 000, 00E, or 00. In particular, such capsules have inner lengths of less than 30 mm, such as less than 26.1 mm, less than 26 mm, or less than 25.3 such as less than 23.4 mm. Accordingly, the width of the foil, which relates to the length of the capsule, is less than 30 mm, such as less than 26.1 mm, less than 26 mm, or less than 25.3 mm such as less than 23.4 mm. Thus, in one embodiment of the present disclosure, the width of the foil is less than 30 mm. However, foils having a smaller width limit the amount of pharmaceutical composition that can be loaded onto the foil. Furthermore, a narrow foil is more likely to unroll to an undesired position, such as not having its convex surface in contact with the mucus lining the intestinal wall. Thus, in a preferred embodiment of the present disclosure, the width of the foil is 8 to 26 mm.

The length of the foil is defined by the diameter of the designated intestine. The foils of the present invention should, when unrolled in the designated intestine, line the inner wall of the intestine covering at least half the inner circumference. Accordingly, in one embodiment of the present disclosure, the designated intestine is the small intestine and the length of the foil is at least 3.9 cm. In another embodiment, the designated intestine is the large intestine and the length of the foil is at least 7.5 cm. The foils of the present disclosure may line substantially the whole circumference of the designated intestine when unrolled in the designated intestine. Thus, in one embodiment of the present disclosure, the designated intestine is the small intestine and the length of the foil is 6.0 - 10 cm, such as 7.8 cm. In yet another embodiment, the designated intestine is the large intestine, and the length of the foil is 12 - 18 cm, such as 15 cm.

The inner wall of the intestine comprises multiple folds. Nevertheless, it is at a macroscopic level substantially concave. Thus, good surface contact between the mucus layer lining the inner wall of the intestine and the surface of the foil unrolled to the diameter of the designated intestine is achieved if the surface of the foil unrolled to the diameter of the designated intestine is convex. Furthermore, good absorption of pharmaceutical composition is achieved if the pharmaceutical composition is comprised on the convex surface of the foil. In its rolled conformation, this convex surface of the rolled foil corresponds to the outer surface. Thus, in a preferred embodiment of the present disclosure, the outer surface of the rolled foil corresponds to the convex surface of the foil.

One embodiment of the present disclosure provides for a foil having a thickness between 100 and 1200 µm. In one embodiment of the present disclosure, the foil has a thickness of 100 to 200 µm, such as 200 to 300 µm, such as 300 to 400 µm, such as 400 to 500 µm, such as 500 to 600 µm, such as 600 to 700 µm, such as 700 to 800 µm, such as 800 to 900 µm, such as 900 to 1000 µm, such as 1000 to 1100 µm, such as 1100 to 1200 µm. In one embodiment, the foil has a thickness of approximately 500 µm. In one embodiment of the disclosure, the foil has a thickness of approximately 700 µm or approximately 800 µm.

### Pharmaceutical composition

Many compounds, such as peptide drugs, are degraded in the intestinal lumen. The present disclosure relates to a rolled foil which comprises a pharmaceutical composition on its outer, convex surface. As the foil unrolls, it presses itself against the intestinal wall and releases the pharmaceutical composition in the confined space between the convex surface of the foil and the intestinal wall. Thus, the exposure of the pharmaceutical composition to the intestinal luminal fluids is minimal. The foils of the present disclosure are therefore especially suited for delivery of drugs which are prone to degradation in the intestinal lumen. Accordingly, in one embodiment of the present disclosure, the pharmaceutical composition comprises one or more ingredients, such as therapeutically active compounds, which are prone to degradation in the intestinal lumen.

Many therapeutically active compounds, such as drugs, have low permeability through the intestinal wall. This can be due to for instance the nature of the therapeutically active compound itself, or due to the mucus layer lining the intestinal wall. Because the foil of the present disclosure presses its convex surface against the intestinal wall, the volume of the space between the convex surface of the foil and the intestinal wall is small compared to the volume of the lumen. Thus, the pharmaceutical composition released into this confined space results in a high local concentration, and the concentration remains high because the pharmaceutical composition is not diluted into the bulk of the luminal fluids. This facilitates absorption of the components of the pharmaceutical composition, even components with low permeability. Thus, in one embodiment of the present disclosure, the pharmaceutical composition comprises a low-permeability drug.

Many polypeptides are prone to degradation in the intestine, in part due to the presence of digestive enzymes in the lumen. The rolled foils of the present disclosure are especially useful in the delivery of polypeptides because the foil of the present disclosure functions as a barrier between the released polypeptide and the digestive enzymes of the intestinal lumen. Thus, in an embodiment of the present disclosure, the pharmaceutical composition comprises a polypeptide. In a further embodiment of the present disclosure, the polypeptide is insulin or analogues thereof.

The mucus layer lining the intestinal wall comprises many different microbes. The foils of the present disclosure are considered useful in the delivery of microbes for seeding into the intestinal mucosa. Thus, in one embodiment of the present disclosure, the pharmaceutical composition comprises probiotic microbes.

The foils of the present disclosure effect a high local concentration of pharmaceutical composition in the space between the convex surface of the foil and the intestinal wall. This is because the foils of the present disclosure provide for a unidirectional release aimed specifically at the intestinal epithelium, rather than at the intestinal lumen in general. Thus, the foil of the present disclosure is considered especially useful for the delivery of therapeutically active compounds using doses that are lower than what would usually be employed, had the therapeutically active compound been administered using a simple, oral formulation.

In one embodiment of the present disclosure, the pharmaceutical composition is flexible to allow for the foil to be rolled and unrolled.

### Covering layer

The rolled foils of the present disclosure are covered in a covering layer. The role of the covering layer is to prevent the rolled foil from unrolling before it reaches its designated intestine. Thus, in a preferred embodiment of the present disclosure, the rolled foil is covered in a covering layer.

A gelatin capsule is one such type of covering layer, which is considered useful in the present disclosure. Gelatin capsules have adequate structural strength to prevent unrolling of the rolled foils of the present disclosure. The capsules can also dissolve and/or melt to release the rolled foil. Thus, in on embodiment of the present disclosure, the covering layer comprises a gelatin capsule. Other structures are also able to prevent unrolling of the rolled foil. Such structures comprise bands or clamps, provided the structures are made of a material that is able to release the rolled foil in the designated intestine.

In one embodiment of the present disclosure, the covering layer comprises a hydroxypropyl methylcellulose (HMPC) capsule. In one embodiment of the present disclosure, the covering layer comprises an enteric capsule. In one embodiment of the present disclosure, the covering layer comprises a pullulan capsule.

In one embodiment of the present disclosure, the covering layer dissolves readily after the enteric coating is removed.

The present disclosure further relates to covering layers which dissolve upon reaching the designated intestine. Release at a specific location is controlled by employing enteric coatings. Such coatings are stable in the acidic environment of the stomach, but dissolve in the less acidic environment of the designated intestine. Thus, in one embodiment of the present disclosure, the covering layer comprises an enteric coating. The pH of the lumen changes throughout the gastrointestinal tract. Thus, highly localised dissolution of the covering layer and unrolling of the foil is achieved by employing enteric coatings that dissolve at specific pH. Thus, in one embodiment of the present disclosure, the enteric coating dissolves in the duodenum. In another embodiment of the present disclosure, the enteric coating dissolves in the jejunum. In yet another embodiment of the present disclosure, the enteric coating dissolves in the ileum. In an even further embodiment, the enteric coating dissolves in the colon. In yet a further embodiment, colonic targeting is achieved by a combination of pH-dependent coatings and pH-independent coatings.

In one embodiment of the present disclosure, the covering layer comprises a size 000 capsule, a size 00E capsule, or a size 00 capsule. In a preferred embodiment, the covering layer comprises a size 00 capsule.

### Radial pressure

In one embodiment of the present disclosure, the foils of the disclosure are produced in a fully unrolled conformation and then rolled. This ensures that the rolled foils possess a certain amount of stress, which facilitates that the foils unroll in the designated intestine. The foils of the present disclosure do not necessarily unroll fully in the designated intestine. In particular, as the foil unrolls to the diameter of the designated intestine and establishes contact with the mucus lining the intestinal wall, the foil still possesses some unrelaxed stress so that contact is maintained between the foil and the mucus lining the inner wall of the designated intestine. The pressure that the foil exerts on the intestinal wall is mediated by the radial force exerted by the foil. This radial pressure is defined as the force per contact area between the foil and the mucus layer lining the intestinal epithelium. The contact area between the foil and the mucus is also termed the effective contact area herein. The radial pressure acts outwards, away from the convex surface of the foil. The foils of the present disclosure do not necessarily exert a uniform radial pressure on the intestinal wall. This is in part due to the fact that the intestinal wall is uneven due to structures such as folds and villi, and in part because the foil might not exert a uniform pressure due to its shape. Peristalsis of the intestine also contributes to the non-uniformity of the radial pressure. Thus, in one embodiment, radial pressure relates to the average pressure exerted by the foil on the mucus lining the intestinal wall. In another embodiment, radial pressure relates to the pressure exerted by part of the foil on the intestinal wall.

As described herein, the foils of the disclosure comprises surface microstructures. Such structures will affect the contact area between the foil and the mucus layer lining the intestinal wall. Thus, in assessing the radial pressure of the foil, the contact area of the foil and mucus layer lining the intestinal wall may be smaller than the bulk surface area of the foil. Thus, in one embodiment of the present disclosure, the radial pressure exerted by the foil on the intestinal wall is assessed based on the surface area of the surface microstructures.

The majority of the gastrointestinal epithelium is covered by mucus. The small intestine is covered by a single layer of mucus, whereas the large intestine is covered by two layers of mucus. The mucus is a viscoelastic translucent aqueous gel that is secreted throughout the gastrointestinal tract, acting as a protective layer and a mechanical barrier. Mucus is a constantly changing mix of many secretions and exfoliated epithelial cells. It has a large water content (≈ 95%). Many particles are prevented from passively diffusing through the mucus layer due to the presence of an internal mesh network. However, the mucus layer can be deformed. Thus, in a preferred embodiment of the disclosure, the radial pressure is sufficient for contacting the mucus layer. In another embodiment, the radial pressure is sufficient for contacting and deforming the mucus layer. In yet another embodiment, the radial pressure is sufficient to let the foil through the mucosal layer such that the foil contacts the epithelial cell layer.

### Foil material

The material of the foil is able to support elastic deformation such that the rolled foil unrolls. In one embodiment of the present disclosure, the material supports elastic deformation at strains up to 50 % when rolled to a rolled foil. The maximum strain in the bulk material is experienced on the convex and concave surfaces of the foil. Furthermore, materials stored may undergo creep. By creep is meant the temporal increase in strain under the influence of a constant stress. Creep and plastic deformation/permanent deformation are not synonymous but creep can result in a permanent deformation. Pharmaceutical formulations may be stored for several months to years before being administered. Thus, in one embodiment of the present disclosure, the rolled foil is made of a material that retains a substantial amount of its elastic deformation over a period of 1 month. In another embodiment of the present disclosure, the rolled foil is made of a material that retains a substantial amount of its elastic deformation over a period of a few months. In a further embodiment of the present disclosure, the material retains a substantial amount of its elastic deformation over a period of several months.

The foils of the present disclosure are preferably made of a biocompatible material. Examples of such materials are biorubbers and elastomers. A further example of an elastomer is a thermoplastic elastomer. It is further advantageous if the foil is made of an FDA-approved material, or a material approved by a similar regulatory body. Thus, in one embodiment of the present disclosure, the foil is made of a biorubber. In another embodiment, the foil is made of an elastomer. In a further embodiment, the foil is made of a thermoplastic elastomer. In yet another embodiment, the foil is made of a material approved for internal medical use.

It is advantageous that the foil is made of a biodegradable material such that it degrades after having released the pharmaceutical composition, which facilitates excretion of the foil. The specific time it takes for a foil to degrade should be tailored to the dosing requirements of the pharmaceutical composition. For instance, if a pharmaceutical composition is to be delivered daily, the foils should degrade within a similar time interval. However, for pharmaceutical compositions being released over several days, the foils are more stable, degrading over several days. Alternatively, the release of the pharmaceutical composition is fast, but the plasma half-life of the active compound is long. In this case, a fast degrading foil can be used with a low administration frequency. For single-time dosage regimes, it is acceptable that the foil is degraded over several weeks. Thus, in a preferred embodiment of the present disclosure, the foil degrades after having released the pharmaceutical composition. The foil may also be made of a non-degradable material, which nevertheless may be excreted without having been degraded. Thus, in one embodiment, the foil is made of a non-biodegradable material. In a specific embodiment of the present disclosure, the non-biodegradable material is polydimethylsiloxane (PDMS). Due to the peristaltic movement of the intestine and continuous secretion of mucus, foils which are not biodegradable may also be excreted. In one embodiment of the present disclosure, the foil is made of a robust yet biodegradable material, such that the foil does not degrade in the intestine, but is biodegradable after excretion.

In one embodiment of the present disclosure, the foil is made of a material having a Young's modulus of 0.1 to 100 MPa. In one embodiment, the foil is made of a material having a Young's modulus of 0.1 to 0.2 MPa, such as 0.2 to 0.4 MPa, such as 0.4 to 0.6 MPa, such as 0.6 to 0.8 MPa, such as 0.8 to 1 MPa, such as 1 to 2 MPa, such as 2 to 3 MPa, such as 3 to 5 MPa, such as 5 to 7 MPa, such as 7 to 10 MPa, such as 10 to 15 MPa, such as 15 to 25 MPa, such as 25 to 50 MPa, such as 50 to 100 MPa. In one embodiment of the present disclosure, the foil has a Young's modulus of approximately 2 MPa or approximately 3 MPa. The radial pressure exerted by the foil on the intestinal wall is dependent on the properties of the material comprising the foil, and the dimensions of the foil, in particular the thickness of the foil. Thus, foils with a thickness between 100 and 500 µm, such as between 200 and 400 µm, made from a material having a Young's Modulus between 1 and 10 MPa are considered especially useful for the delivery of pharmaceutical compositions to the intestine. Alternatively, foils having a thickness between 100 and 1200 µm, such as between 100 and 1000 µm, made from a material having a Young's modulus between 0.1 and 100 MPa, such as between 1 and 10 MPa, are considered useful for the delivery of pharmaceutical compositions to the intestine. Young's Modulus describes the tendency of a material to deform elastically, and is therefore a suitable way to define the foils of the present disclosure, as they must also deform elastically. Thus, in a preferred embodiment of the present disclosure, the rolled foil is made of a material with a Young's modulus of 1 to 10 MPa and a bulk thickness of 100 to 1200 µm, such as 100 to 1000 µm, such as 100 to 500 µm. It is acknowledged that the radial pressure exerted by the foil may be tuned either via the thickness of the foil or via the Young's modulus of the material. That is, thicker foils made from materials with higher Young's moduli will provide for a higher radial pressure, whereas thinner foils made from materials with low Young's moduli will provide for a lower radial pressure. In particular, thicker materials will provide for higher radial pressures than thinner materials. Likewise, materials with a higher Young's modulus will provide for higher pressures than materials with a lower Young's modulus.

### Microstructures on the foil

The convex surface of the foils of the disclosure comprises microstructures that facilitate delivery, such as optimise absorption or improve conditions for delivery of the pharmaceutical composition. They may improve mucus contact and loading capacity of the pharmaceutical composition. They may also affect the deformation of the foil.

The microscale topography of the foil may for instance be composed of (a) interconnected ridges that form hollow compartments, (b) individual compartments formed by protruding sidewalls or (c) needle-like structures. The surface topography may be made using conventional fabrication techniques, such as casting/moulding, hot embossing, compression injection moulding, micromachining, laser ablation, UV-VIS lithography and/or 3D printing. Said topography is either replicated directly in the foil, constructed on the surface of the foil, embedded in the surface of the foil, or encapsulated in the surface of the foil.

Close-packed compartments may be advantageous, as they allow for higher loading of the pharmaceutical composition. Close-packed compartments may also allow for more facile loading of the pharmaceutical composition. Thus, in one embodiment of the present disclosure, the foil comprises microstructures that are close-packed. In a further embodiment of the present disclosure, the foil comprises microstructures that are compartments which are close-packed.

Thus, in one embodiment of the present disclosure, the convex surface of the foil comprises microstructures. In a further embodiment, the microstructures are ridges that define compartments, such as for instance protruding walls defining compartments. In a further embodiment, the ridges are interconnected. As the foils of the present disclosure are pressed against the mucus layer of the intestinal wall, such surface microstructures will sink into the mucus layer. It is advantageous if such structures are comparable in height to the thickness of the mucus layer, as the foil may better form a tight seal between the foil and the epithelial cells of the intestinal wall, and/or provide for a shorter distance from the microstructures to the epithelium. Thus, in one embodiment of the present disclosure, the ridges are 100 to 300 µm tall. The ridges are able to support their own weight, in addition to any pharmaceutical composition loaded into the compartments. Thus, in one embodiment of the present disclosure, the ridges are 20 to 300 µm wide. In one embodiment of the present disclosure, the height/width aspect ratio (i.e. the ratio of the ridge height to the ridge width) is between 1 and 5. In a particular embodiment, the height/width aspect ratio of the ridges is between 2 and 4.

In an embodiment of the present disclosure, the interconnected ridges define compartments that are hexagonal. Such interconnected ridges may improve the robustness of the foils and/or the microscale ridges.

The microstructures on the convex surface of the foils may comprise needles or needle-like structures. Such needles are especially advantageous as they can penetrate the mucus layer, and optionally the epithelial cells of the intestinal wall as well. The mucus layer of the small intestine is approximately 100 µm thick. Thus, the needle microstructures are for instance up to 100 µm tall. Alternatively, the needles are longer than the thickness of the mucus layer so that they may penetrate the epithelium. Accordingly, in one embodiment of the disclosure, the surface structures comprise needles. In a further embodiment, the needles are 10 to 100 µm tall. In an even further embodiment, the surface microstructures comprise needles that are 100 to 300 µm tall.

The surface microstructures of the present invention may be walls that are perpendicular to the convex foil surface. Such shape may provide for especially robust microstructures. Alternatively, the surface structures may be tapered such that they are wider at the base of the structure than at the top of the structures. Such structures may be advantageous for allowing the microstructures to sink into the mucosal layer of the designated intestine. The most protruding parts of the microstructures, i.e. the top of the microstructures, may be flat, rounded, or acute.

In one embodiment of the present disclosure, the microstructures are comprised of the same material as the bulk foil. In another embodiment, the microstructures are comprised of a material different than the bulk foil. In the latter case, the material that the microstructures are comprised of may possess additional desired properties. Such properties may relate to mucoadhesion and biocompatibility.

The foils of the present disclosure exerts a force on the mucus lining the intestine. The radial pressure is given as this force divided by the area of contact between the foil and the mucus lining the intestine. As the foil of the present disclosure unrolls, protruding microstructures present will contact the intestinal mucus layer. The contact surface area between the microstructures of the unrolling foil and the mucus layer of the intestine is at this time small, and thus, the corresponding radial pressure exerted on the mucus layer is relatively high. However, as the microstructures of the foil sink into the mucus layer of the intestine, the mucus layer will make contact with the bulk foil material. The contact area between the mucus layer and the bulk foil material is higher than that of the area of the microstructures. The radial pressure exerted on the mucus layer thus decreases at this point during the unrolling process, and the microstructures will optionally refrain from sinking further into the mucus layer and/or refrain from penetrating the epithelial cell layer of the intestinal wall.

Because the surface area of the microstructures is less than the bulk surface area of the foil, less force is needed in order for the microstructures to sink into the mucus layer. Thus, the force exerted by the foil when it unrolls can be lower than for foils comprising no surface microstructures, and a material with a reduced Young's modulus can be employed. Therefore, in one embodiment of the present disclosure, the foil comprises microstructures and is made of a material that has a reduced Young's modulus.

### Loading of pharmaceutical composition

The foil of the present disclosure comprises a pharmaceutical composition. The pharmaceutical composition may be introduced directly on the foil as a layer covering the convex surface of the foil. Alternatively, the pharmaceutical composition is incorporated into the bulk foil material. After unrolling of the foil in the designated intestine, the pharmaceutical composition will diffuse out of the foil material. Thus, in one embodiment of the present disclosure, the pharmaceutical composition is present on the foil as a layer covering the convex surface of the foil. In another embodiment of the present disclosure, the pharmaceutical composition is incorporated into the bulk material of the foil, and will diffuse out of the foil after the foil unrolls in the designated intestine. Alternatively, the foil material is biodegradable, or part of the foil material is degradable, and the pharmaceutical composition is released as the foil degrades.

The pharmaceutical composition may be loaded onto the full surface of the foil, or onto only parts of the surface of the foil. In one embodiment of the present disclosure, the foil comprises the pharmaceutical composition on substantially its entire surface. In one embodiment of the present disclosure, the foil comprises the pharmaceutical composition on only parts of its surface. In a specific embodiment of the present disclosure, the foil does not comprise the pharmaceutical composition on the surface constituting the innermost winding of the rolled foil. In a further embodiment of the present disclosure, the foil does not comprise the pharmaceutical composition on the surface constituting the innermost winding of the rolled foil, but the foil does comprise the pharmaceutical composition on the surface constitution the windings beyond the innermost winding, such as the surface constituting the second winding, the surface constituting the third winding, etc., until and including the surface constituting the outermost winding.

The pharmaceutical composition can be incorporated in the bulk foil material such that it is evenly dispersed throughout the bulk foil material. It is advantageous that the pharmaceutical composition primarily diffuses out of the foil through the convex, outer surface rather than the inner, concave surface, such that the majority of the pharmaceutical composition is exposed to the epithelial cells of the intestinal wall. Diffusion of the pharmaceutical composition out of the inner, concave surface of the foil can be prevented by covering the inner, concave surface of the foil with a material through which the pharmaceutical composition cannot diffuse. Alternatively, the pharmaceutical composition is incorporated in the bulk foil such that it is non-uniformly dispersed, namely such that the majority of the pharmaceutical composition is situated closest to the outer, convex surface. Both these strategies will have the effect that more of the pharmaceutical composition diffuses out of the outer, convex surface than out of the inner, concave surface. Thus, in one embodiment of the present disclosure, the pharmaceutical composition is incorporated into the bulk foil material wherein the foil further comprises an impermeable layer covering the inner, concave surface of the foil, wherein the impermeable layer covering the inner, concave surface of the foil is impermeable to one or more of the components of the pharmaceutical composition. In another embodiment of the present disclosure, the pharmaceutical composition is incorporated into the bulk foil material wherein the majority of the pharmaceutical composition is situated closer to the convex, outer surface of the foil than to the concave, inner surface of the foil.

The pharmaceutical composition may alternatively be released from the foil because the foil material degrades. It is advantageous that the pharmaceutical composition is released primarily at the convex, outer surface rather than the inner, concave surface, such that the majority of the pharmaceutical composition is exposed to the epithelial cells of the intestinal wall. Release of the pharmaceutical composition out of the inner, concave surface of the foil can be prevented by covering the inner, concave surface of the foil with a material that does not degrade, or degrade slower than the material comprising the convex surface. Alternatively, the pharmaceutical composition is incorporated in the bulk foil such that it is non-uniformly dispersed, namely such that the majority of the pharmaceutical composition is situated closest to the outer, convex surface. Both these strategies will have the effect that more of the pharmaceutical composition is released from the outer, convex surface than out of the inner, concave surface. Thus, in one embodiment of the present disclosure, the pharmaceutical composition is incorporated into the biodegradable bulk foil material wherein the foil further comprises an impermeable or slower-biodegrading layer covering the inner, concave surface of the foil. In another embodiment of the present disclosure, the pharmaceutical composition is incorporated into the biodegradable bulk foil material wherein the majority of the pharmaceutical composition is situated closer to the convex, outer surface of the foil than to the concave, inner surface of the foil.

The pharmaceutical composition of the present disclosure may also be loaded into the compartments defined by the surface microstructures. It is advantageous to load the pharmaceutical composition into the surface microstructures, as this ensures a high loading capacity of the foils. The compartments are likewise considered useful as they facilitate loading of a plurality of different pharmaceutical compositions into the same foil. Furthermore, the compartments facilitate precise loading of the foils. Thus, in one embodiment of the present disclosure, the pharmaceutical composition is situated in the compartments.

The pharmaceutical composition may be situated on the ridges of the surface microstructures, such as loaded on or into the ridges or needles, or be incorporated into the material from which the ridges or needles are formed. Upon unrolling of the foil in the designated intestine, the ridges or needles make contact with the mucus layer and sink into the mucus layer, optionally making contact with the epithelial cells of the intestinal wall, and further optionally penetrating into the wall of the intestine. Thus, incorporating the pharmaceutical composition into or onto the surface microstructure facilitates proximity between the pharmaceutical composition and the epithelial cells of the intestinal wall. Thus, in one embodiment of the present disclosure, the pharmaceutical composition is situated on the foil surface microstructures. In another embodiment, the pharmaceutical composition is incorporated into the material making up the surface microstructures of the foil. Particularly, in one embodiment of the present disclosure, the pharmaceutical composition is situated on the ridges of the foil surface microstructures. In another embodiment, the pharmaceutical composition is incorporated into the material making up the ridges of the foil surface microstructures. In yet another embodiment of the present disclosure, the pharmaceutical composition is situated on the needles of the foil surface microstructures. In yet another embodiment of the present disclosure, the pharmaceutical composition is incorporated into the material making up the needles of the foil surface microstructures.

The pharmaceutical composition may alternatively be incorporated into compartments within the bulk foil material or within surface microstructures. This type of loading can facilitate high loading capacity with slow and/or extended release. Thus, in one embodiment of the present disclosure, the pharmaceutical composition is situated in compartments within the bulk foil material. In another embodiment, the pharmaceutical composition is situated in compartments within the surface microstructures.

The different embodiments described above possess different advantages with respect to delivery of therapeutically active compounds, excipients, adjuvants, etc. For instance, it may be advantageous to incorporate permeation enhancers on the surface of the microstructures, whereas therapeutically active compounds would be incorporated into the material of the surface structures. Thus, in one embodiment of the present disclosure, the different components of the pharmaceutical composition are situated in or on different parts of the bulk foil material and/or the foil microstructures.

### Designated intestine

The rolled foils of the present disclosure can be used for targeted delivery of a pharmaceutical composition to any part of the gastrointestinal tract, in particular the small intestine and the large intestine, preferably the small intestine. The radial pressure exerted by the foil on the designated intestine depends on the diameter to which the foil has unrolled, which in turn depends on the diameter of the designated intestine. Thus, in one embodiment of the present disclosure, the foil unrolls to the diameter of the designated intestine. In a further embodiment, the radial pressure exerted by the foil on the designated intestine depends on the diameter of the designated intestine.

The rolled foil may be used for targeted delivery of a pharmaceutical composition to the small intestine, i.e., the designated intestine is the small intestine. The small intestine has an inner diameter of approximately 2.5 cm. Upon administration of a rolled foil to the small intestine, the foil unrolls to a diameter of approximately 2.5 cm. Thus, in an embodiment of the present disclosure, the foil exerts a radial pressure when unrolled to a diameter of 2.0 to 3.0 cm. In a preferred embodiment, the foil exerts a radial pressure when unrolled to a diameter of 2.5 cm.

The rolled foil may be used for targeted delivery of a pharmaceutical composition to the large intestine, i.e., the designated intestine is the large intestine. The large intestine has an inner diameter of approximately 4.8 cm. Upon administration of a rolled foil to the large intestine, the foil unrolls to a diameter of approximately 4.8 cm. Thus, in an embodiment of the present disclosure, the foil exerts a radial pressure when unrolled to a diameter of 4.0 to 6.0 cm. In a preferred embodiment, the foil exerts a radial pressure when unrolled to a diameter of 4.8 cm.

The sizes of the intestines vary between individuals, and depend on factors such as age, lifestyle, and body weight. For instance, children will typically have narrower intestines than adults. Likewise, body weight may correlate with the diameter of the intestines. Thus, in one embodiment of the present disclosure, the designated intestine is the small intestine having an inner diameter of more than 2.5 cm. In another embodiment, the designated intestine is the small intestine having a diameter of less than 2.5 cm. In yet another embodiment of the present disclosure, the designated intestine is the large intestine having a diameter of more than 4.8 cm. In yet another embodiment, the designated intestine is the large intestine having a diameter of less than 4.8 cm.

The rolled foils of the present disclosure are considered useful for delivery of a pharmaceutical composition to the intestine because the foil contacts intimately with the intestinal wall while allowing for free passage of the luminal fluids while the foil delivers the pharmaceutical composition to the intestinal wall. This is demonstrated in the examples disclosed herein. Other drug delivery platforms capable of delivery to the intestines exist, however, these may take up substantial space in the intestine, which may hinder the passage of the luminal fluids.

### Sealing layer

The rolled foil comprising the pharmaceutical composition may be sealed with a sealing layer which affects the release profile of the pharmaceutical composition. Any such sealing layer may be applied to the entire foil or to only parts of the foil. For instance, such sealing layer may cover the entire foil because it is feasible during manufacture. Alternatively, it may cover only the convex surface of the foil. Another option is to cover only the compartments of the microstructures such as to seal the pharmaceutical composition inside. Yet a further option is to cover only the needle microstructures of the foil. Thus, in one embodiment of the present disclosure, the rolled foil comprising the pharmaceutical composition is sealed with a sealing layer. In a further embodiment, said sealing layer is 10 to 50 µm thick. In one embodiment of the present disclosure, the sealing layer covers the entire rolled foil. In another embodiment, the sealing layer covers only the convex surface of the foil. In yet another embodiment of the present disclosure, the sealing layer covers only parts of the convex surface of the foil.

The sealing layer can be comprised of a composition which promotes passage through the mucus layer of the pharmaceutical composition. Examples of such compositions are gel-sol promoters. Gel-sol promoters induce a change in the intestinal mucus layer by liquefying the mucus mesh network. This facilitates that the pharmaceutical composition can more easily diffuse through the mucus layer, and that the surface microstructure may more easily sink into the mucus layer. Thus, in one embodiment of the present disclosure, the sealing layer comprises a composition which promotes mucus penetration.

The sealing layer can be comprised of a composition which reduces the rate of release of the pharmaceutical composition. Examples of such reagents are extended-release agents, sustained-release agents, or controlled-release agents. It can be especially advantageous to cover a foil having a high content of pharmaceutical composition with an extended-release sealing layer such that the foils can be administered more infrequently. Thus, in one embodiment of the present disclosure, the rolled foil comprises a sealing layer that comprises an extended-release agent. In another embodiment, the rolled foil comprises a sealing layer that comprises a sustained-release agent. In yet another embodiment, the rolled foil comprises a sealing layer which comprises a controlled-release agent.

The sealing layer can be comprised of a composition which delays the release of the pharmaceutical composition. Thus, in one embodiment of the present disclosure, the sealing layer comprises a delayed-release agent.

The sealing layer can comprise an enteric coating. The dissolution of an enteric coating is triggered by changes in the environment surrounding the enteric coating, such as for instance changes in the acidity of the environment. Thus, in one embodiment of the present disclosure, the sealing layer comprises an enteric coating.

The sealing layer may be covering the full surface of the foil, or only parts of the surface of the foil. In one embodiment of the present disclosure, the foil comprises the sealing layer on substantially its entire surface. In one embodiment of the present disclosure, the foil comprises the sealing layer on only parts of its surface. In a specific embodiment of the present disclosure, the foil does not comprise the sealing layer on the surface constituting the innermost winding of the rolled foil. In a further embodiment of the present disclosure, the foil does not comprise the sealing layer on the surface constituting the innermost winding of the rolled foil, but the foil does comprise the sealing layer on the surface constitution the windings beyond the innermost winding, such as the surface constituting the second winding, the surface constituting the third winding, etc., until and including the surface constituting the outermost winding.

In one embodiment of the present disclosure, the sealing layer is flexible to allow for the foil to be rolled and unrolled.

### Method of manufacturing

The rolled foils of the present disclosure can be produced by first loading a foil with a pharmaceutical composition. The foil comprises surface microstructures which the pharmaceutical composition may be loaded into or onto. The foil may also be produced with the pharmaceutical composition already incorporated. Following production of a foil incorporating a pharmaceutical composition, the foil is optionally sealed with a sealing layer. The foil is then rolled, producing a rolled foil, which is further covered in a covering layer. Thus, one embodiment of the present disclosure provides for a method of manufacturing a rolled foil comprising a pharmaceutical composition comprising the steps of:
a. providing a foil that exerts a radial pressure when rolled to a diameter of a designated intestine wherein the foil has a length of at least half the circumference of the designated intestine,
b. loading the foil with the pharmaceutical composition, and optionally applying a sealing layer,
c. rolling the foil, and
d. covering the rolled foil in a covering layer.

### Method of administration

The rolled foils of the present disclosure may be administered orally. Thus, one embodiment of the present disclosure provides for a method of administering a pharmaceutical composition to a designated intestine comprising the steps of:
a. providing a rolled foil having on the convex surface a pharmaceutical composition, wherein the rolled foil having on the convex surface a pharmaceutical composition is comprised inside a covering layer, wherein the rolled foil exerts a radial pressure when unrolled to a diameter of a designated intestine and wherein the length of the rolled foil is at least half the length of the inner circumference of the designated intestine, and
b. administering the rolled, covered foil orally to a subject in need thereof.

### Examples

### Example 1: Fabrication of hexagonal patterned silicon master

A SiO₂ hard mask was used for the deep anisotropic etch into the silicon (Si) substrate. A 1 µm thick wet thermal oxide was grown (Tempress horizontal furnace) and masked using the positive photoresist AZ^{®} 5214 E (MicroChemicals, Ulm, Germany). In order to increase resist adhesion, hexamethyldisilizane was deposited as part of the procedure for spin coating the 1.5 µm thick resist layer. The UV-exposure was conducted using an MLA100 Tabletop Maskless Aligner (Heidelberg Instruments, Heidelberg, Germany) and a dose of 90 mJ/cm² before developing the exposed pattern for 90 s using tetramethylammonium hydroxide. Subsequently, the hexagonal pattern was transferred into the oxide layer using an Advanced Oxide Etcher (STS MESC Multiplex ICP) with C₄F₈ and H₂ as the reactive gasses. The remaining resist mask was stripped using a combination of energetic oxygen plasma in a barrel asher (300 Semi Auto Plasma Processor, PVA TePla, Wettenberg, Germany) and submersion into 7-up at 80 °C. The 7-up consisted of concentrated H₂SO₄ with (NH₄)₂S₂O₈ salt added just prior to immersion into the solution that effectively stripped reaining traces of resist. The honeycomb structures were then etched approximately 130 µm into the Si using an inductively coupled plasma deep reactive ion etching tool (STS Pegasus, SPTS Technologies, Orbotech, Yavne, Israel). The tool utilized a Bosch-type process for performing deep anisotropic etching of Si by alternating between Si etching using SF₆ and O₂ and sidewall passivation obtained by deposition of C₄F₈. The substrate temperature was kept at 0 °C throughout the etching process and the substrate was cleaned using oxygen plasma and a mixture of concentrated H₂SO₄ and H₂O₂ (4:1 v/v, commonly referred to as piranha) before stripping the remaining oxide mask in aqueous buffered hydrofluoric acid (12%, v/v) with NH₄F. As the Si-master was intended for foil fabrication by means of casting with the elastomer polydimethylsiloxane (PDMS), an anti-stick coating was deposited by molecular vapor deposition (MVD 100 Molecular Vapor Deposition System, Applied Microstructures, Orbotech, Yavne, Israel). This effectively created a monolayer of 1H,1H,2H,2H-perflourodecyltrichlorosilane which decreased the surface energy of the Si-master. This in turn promoted demolding of the delicate honeycomb protrusions after the PDMS casting and ensured that elastomer rip-off was prevented, thereby preserving the pristine master for multiple replication cycles. The width and depth of the hexagonal trenches were measured as 46 µm and 127 µm, respectively, by vertical scanning interferometry using a PLu Neox 3D Optical Profiler (Sensofar Metrology, Terrassa, Spain).

### Example 2: Fabrication of PDMS foil

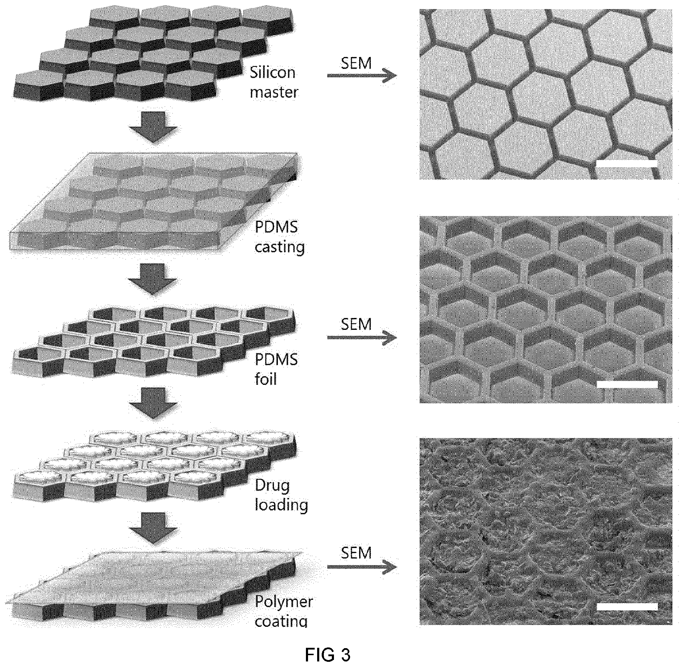

The self-unfolding (i.e. self-unrolling) elastomer foils were fabricated by casting with Sylgard^{™} 184, which is a two-component product consisting of a PDMS base and a curing agent. The base resin and curing agent were mixed in a 10:1 ratio (w/w) and degassed in a desiccator for 30 min prior to use. The mixture was then poured onto the silicon master and placed in a desiccator once more in order to remove potential air bubbles, which would otherwise compromise the structure replication fidelity. The uncured PDMS replica was then kept in an oven at 37 °C overnight and the cured elastomer foil was subsequently peeled from the Si-master. This resulted in thin foils with a length of 7 mm with a well-defined surface topography composed of protrusions arranged in a honeycomb pattern with a height of 125 µm. The topography of the foils was characterized using vertical scanning interferometry on a PLu Neox 3D Optical Profiler (Sensofar Metrology, Terrassa, Spain), The fabrication steps of the PDMS foil is outlined in part of Figure 3.

### Example 3: Loading of the foils and capsule preparation

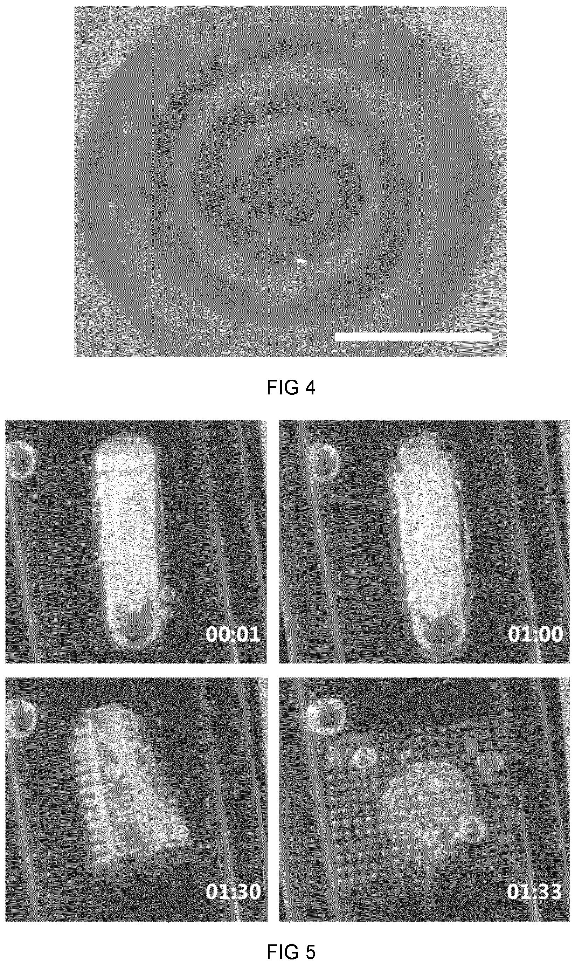

PDMS foils were loaded with a powder mixture of insulin, sodium dodecyl sulfate (SDS) as permeation enhancer, and soybean trypsin inhibitor (5:3:2, w/w/w) by tapping the powder on top of the foil and gently scraping off any excess amount. A solution of Eudragit^{®} L 100 (1%, w/v) and dibutyl sebacate (0.1%, w/v) in isopropanol (IPA) was then spray coated on top of the hexagonal openings to seal the powder inside thus enabling subsequent handling of the foils by forming a sealing layer. An ExactaCoat Ultrasonic Spray System (Sono-Tek, Milton, NY, USA) was used for this purpose with an infuse rate of 0.05 mL/min, path speed of 5 mm/s and shaping air pressure of 0.02 mbar. The generator power was set to 2.2 W and an AccuMist nozzle was applied with a sample-to-nozzle distance of 50 mm. The loaded foils are shown in Figure 3. Immediately after spray coating, the foils were gently rolled between the finger tips such that they comprised 2-3 turns. The foils were then inserted into size 9 gelatin capsules (Torpac, Fairfield, NJ, USA). A flat Si-chip was additionally coated to determine the coating thickness by scanning electron microscopy using a Hitachi TM3030 Plus tabletop microscope (Hitachi High-Technologies Europe, Krefeld, Germany) with an accelerating voltage of 15 kV. Moreover, the capsules for *in vivo* studies were loaded with a cylindrical (2 × 1 mm) power magnet (Magnetz, Hvidovre, Denmark) before locking the capsule cap. Enteric coating of the capsules was carried out by two dip coating cycles, first dipping the capsule body and afterwards the cap in Eudragit^{®} L 100-55 (15%, w/v) and dibutyl sebacate (0.75%, w/v) in IPA. Figure 4 shows the rolled foil inside the capsule.

### Example 4: In vitro release of insulin

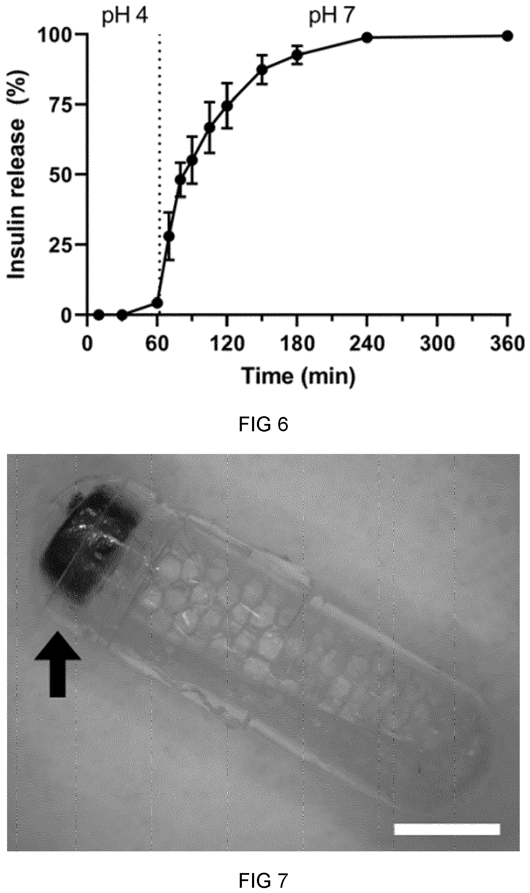

A 400-DS Apparatus 7 (Agilent Technologies, Santa Clara, CA, USA) was installed with 5 mL sample cells and supplied with a 50 mM citrate buffer at pH 4 and a 50 mM phosphate buffer at pH 7. The enteric coated capsules were placed in 50-mesh basket sample holders and dissolution carried out at 37 °C for 1 h at pH 4 followed by 5 h at pH 7 with a dip speed of 15 DPM. Automatic sampling of 0.5 mL was timed at 10, 30, 60, 70, 80, 90, 105, 120, 150, 180, 240 and 360 min, each with partial replacement of dissolution medium except for the sample at 60 min where the citrate buffer was fully replaced with phosphate buffer. Insulin quantification was immediately carried out by reversed phase high performance liquid chromatography on a Dionex Ultimate 3000 system (Thermo Fisher Scientific, Waltham, MA, USA) equipped with a Kinetex XB-C18 column (100 × 4.6 mm, 5 µm, 100 Å; Phenomenex, Torrance, CA, USA). The following gradient of mobile phase A: 0.1% (v/v) TFA in water and B: 0.1% (v/v) TFA in acetonitrile was applied with a flow rate of 0.5 mL/min: 0 - 6.0 min A:B (77:23 to 50:50, v/v), 6.0 - 6.5 min A:B (50:50 to 77:23, v/v) and 6.5 - 8.0 min A:B (77:23, v/v). Insulin was quantified as the area under the curve of the UV-absorbance peak at 276 nm against a standard curve from 10 - 1000 µg/mL with an injection volume of 20 µL and column temperature of 22 °C. The average amount of insulin when loading the foil with insulin, SDS and STI (5:3:2 w/w/w) was determined to be 606 µg, corresponding to 17.5 units. The release profile is shown in Figure 6.

### Example 5: In vivo absorption of insulin

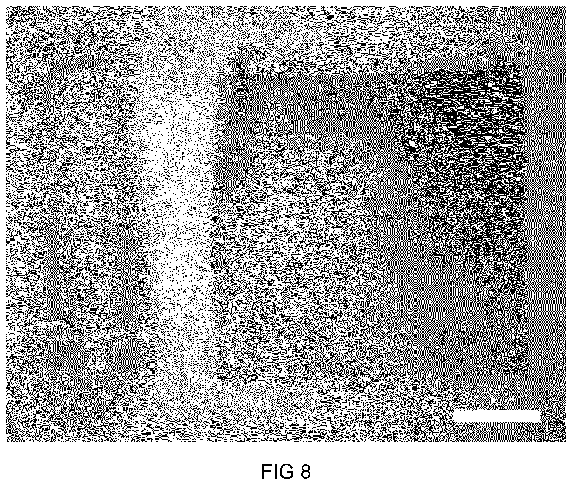

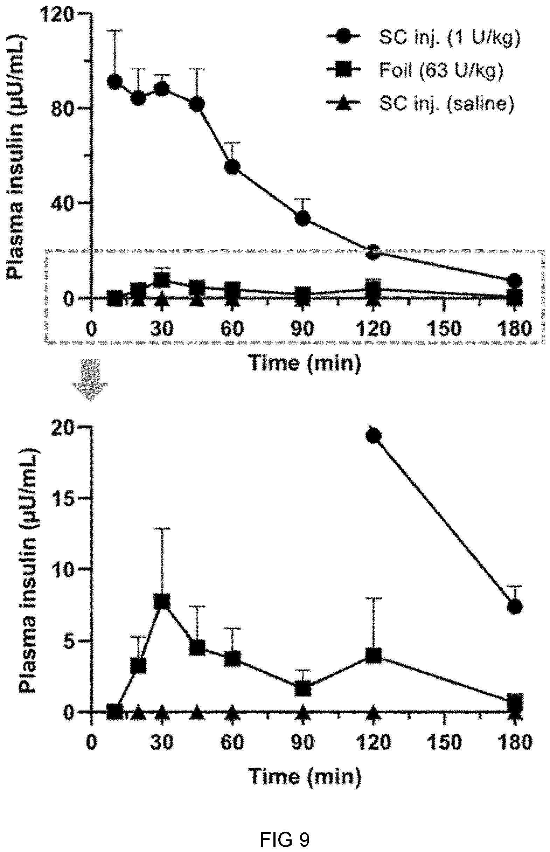

In total, 11 male Sprague Dawley rats (Janvier Labs, Le Genest-Saint-Isle, France) weighing 255 - 310 g were divided into three groups and housed with reversed day/night rhythm (12/12 h). The rats were fasted with *ad libitum* access to water for 12 - 14 h before initiating the study. Bolus anesthesia of fentanyl (236 µg/kg), fluanisone (7.5 mg/kg) and midazolam (3.75 mg/kg) was given as subcutaneous (SC) injections with repeated SC injections every 30 min of 1/3 of the bolus dose together with 0.4 mL saline. The abdominal cavity was opened to expose the gastrointestinal tract prior to dosing. Two groups, each of three rats, were administered SC injections of insulin (1 U/kg) or saline as positive and negative control, respectively. Five rats were instead administered the enteric coated capsules directly to the stomach by the use of an oral gavage dosing tube. Gastric emptying was then facilitated by dragging the capsules to the duodenum by an external magnet (t = 0 min). Blood sampling of 200 µL was taken from the tail vein into Microvette^{®} 200 K3E tubes (Sarstedt, Nümbrecht, Germany) at 10, 20, 30, 45, 60, 90, 120 and 180 min followed by isolation of the blood plasma by centrifugation at 9,300 × g at 4 °C using a Microcentrifuge 5415 R (Eppendorf, Hamburg, Germany). Plasma samples were stored at -20 °C until insulin quantification was carried out by an enzyme-linked immunosorbent assay as described by the manufacturer (Mercodia, Uppsala, Sweden). The rats were euthanized by intracardiac injections of pentobarbital (100 mg/kg) after the final blood sampling. Figure 7 shows a coated gelatin capsules comprising the loaded foil. Figure 8 shows a foil retrieved after the study. Figure 9 shows the plasma insulin over time for the three groups.

### Example 6: Dynamic unrolling of PDMS foil inside pig small intestine

A perfusion setup was employed to assess ex *vivo* unrolling of a foil inside a pig small intestine. A PDMS (Young's modulus = 1-1.5 MPa) foil of length=50 mm, width=15 mm and thickness=700 µm, with the X-ray contrast agent BaSO₄ dispersed in the material, was used. The foil was rolled and stored in a size 0 hard shell gelatin capsule. Upon perfusion of the intestine with a phosphate buffered saline solution, the gelatin capsule dissolved, and the foil started unrolling (see Figure 13 t=02:46). Full unrolling was reached in approximately 10 s at t=02:55. The foil caused a minor local expansion of the intestine as it pressed against the interior of the tissue, evidencing that the foil exerts a pressure on the wall of the intestine. Planar X-ray imaging of the intestine with the unrolled foil further supported that the foil established a close contact with the intestinal wall, while leaving the lumen of the intestine open. The results demonstrate that the dynamic event (i.e. unrolling) is triggered by the dissolution of the covering layer and the fully unrolled conformation is established rather fast (i.e. within 10 s in the experiment.

### Example 7: Surface treatment of PDMS foils

It was found that the adhesion of spray coated sealing layers on PDMS foils could be improved by surface treatment aimed at increasing the surface energy.

The foil was first exposed to UV light in an oxygen rich environment which facilitated oxidation of the surface thereby creating functional SiOₓ groups. The foil was subsequently immersed in 1 wt% polyvinyl alcohol (PVA) dissolved in Milli-Q water. This treatment provided foils having a more hydrophilic surface (i.e. the surface energy was increased). Figure 14 A shows an untreated PDMS foil exhibiting a 110° water contact angle (WCA). Figure 14 B shows a surface-treated PDMS foil with significantly reduced WCA. The WCA is a measure of the surface energy, wherein a high WCA corresponds to a hydrophobic surface having low surface energy, and a low WCA corresponds to a hydrophilic surface having high surface energy. Accordingly, the surface treatment increased the surface energy of the PDMS.

Figures 14 C and 14 D show scanning electron microscopy images of microstructured PDMS foils spray coated with sealing layer. Figure 14 C shows a PDMS foil with no surface treatment. The spray coated sealing layer peels off as a result of low adhesion to the PDMS. Figure 14 D shows a PDMS foil having undergone the surface treatment to increase its surface energy. The spray coated sealing layer remained uniform and intact, even after rolling and unrolling the foil (depicted on the figure). The spray coated layer adheres firmly to the close-packed hexagonal protrusions forming the compartments for loading of pharmaceutical compositions. The increase in adhesion is a direct result of the covalent bonding of PVA to the PDMS foil.

### Example 8: Pressure exerted on the intestinal wall

To investigate the stead-state surface pressure distribution of an unrolled PDMS foil inside the small intestine, finite element analysis (FEA) using Ansys was carried out. Explicitly, the unrolling of a PDMS foil having a thickness of 250 µm, a width of 30 mm, a length of 71 mm, a Young's modulus of 3 MPa and a Poisson's ratio of 0.49 was modelled inside a flexible tube (wall thickness=1 mm, inner diameter=25 mm, Young's modulus=80 kPa) mimicking the mechanical properties of the small intestine via a phenomenological model. The Young's modulus of the cylindrical tube representing the small intestine was estimated based on linear regression to experimental data obtained from planar biaxial pull tests on small intestinal tissue of pigs (see Bellini, C., Glass, P., Sitti, M. & Di Martino, E. S. Biaxial mechanical modeling of the small intestine. J. Mech. Behav. Biomed. Mater. 4, 1727-1740 (2011) for method). In the FEA, it was safely assumed that both the PDMS foil and the flexible tube could be modelled as linearly elastic materials. Furthermore, it was assumed that the steady-state conformation exhibited symmetry around the center point (with respect to length) of the foil and interfacial friction between the foil and the tube was neglected.

From modelling of the steady-state surface pressure distribution, a maximum pressure of approximately 295 Pa was found at the free ends of the foil. The surface pressure was significantly lower (21-33 Pa) when looking at the middle part of the foil (i.e. the part of the foil located halfway between the two ends). These findings support the observation described in example 6: the foil unrolls in the designated intestine and exert a pressure on the intestinal wall thereby ensuring an intimate contact between the convex surface of the foil and the interior of the intestine. In combination with the qualitative ex *vivo* results, this quantitative investigation show, that the foil should not inflict damage on the intestinal tissue. The human small intestine supports a maximum stress of approximately 0.9 MPa. From the quantitative modelling results, it is clear that the exerted maximum pressure cannot be perceived as being harmful to the integrity of the intestinal tissue.

### Example 9: Exemplary foils for delivery of a pharmaceutical composition

It is contemplated that a foil having width 10-20 mm, length 70 mm, thickness 250 µm, and a Young's modulus of 1 MPa will be useful for delivery of a pharmaceutical composition to the small intestine of a human subject. It is contemplated such a foil will exert a maximum pressure on the intestinal wall when unrolled in the small intestine of for example 50-150 Pa.

It is contemplated that a foil having width 10-20 mm, length 70 mm, thickness 500 µm, and a Young's modulus of 3 MPa will be useful for delivery of a pharmaceutical composition to the small intestine of a human subject. It is contemplated such a foil will exert a maximum pressure on the intestinal wall when unrolled in the small intestine of for example 300-900 Pa.

It is contemplated that a foil having width 10-20 mm, length 70 mm, thickness 500 µm, and a Young's modulus of 10 MPa will also be useful for delivery of a pharmaceutical composition to the small intestine of a human subject. It is contemplated such a foil will exert a maximum pressure on the intestinal wall when unrolled in the small intestine of for example 1000-3000 Pa.

It is contemplated that a foil having width 10-20 mm, length 135 mm, thickness 250 µm, and a Young's modulus of 1 MPa will be useful for delivery of a pharmaceutical composition to the large intestine of a human subject. It is contemplated such a foil will exert a maximum pressure on the intestinal wall when unrolled in the large intestine of for example 12.5-37.5 Pa.

It is contemplated that a foil having width 10-20 mm, length 135 mm, thickness 250 µm, and a Young's modulus of 10 MPa will also be useful for delivery of a pharmaceutical composition to the large intestine of a human subject. It is contemplated such a foil will exert a pressure on the intestinal wall when unrolled in the large intestine of for example 125-375 Pa.

## Claims

1. A rolled foil having on the convex surface of the rolled foil a pharmaceutical composition, wherein the rolled foil and the pharmaceutical composition are comprised inside a covering layer, wherein the convex surface of the rolled foil exerts a radial pressure when unrolled to a diameter of a designated intestine, wherein the convex surface comprises microstructures, and wherein the length of the rolled foil is at least half the length of the inner circumference of the designated intestine.

2. The rolled foil according to claim 1, wherein:
a. the rolled foil comprise one or more turns, such as at least two turns, such as at least three turns,
b. the rolled foil has an outer diameter less than 9.55 mm, such as an outer diameter less than 9.40 mm, such as an outer diameter less than 8.25 mm, such as an outer diameter less than 8.00 mm,
c. the width of the foil is less than 30 mm, such as 8 to 26 mm, and/or
d. wherein the foil has a bulk thickness of 100 to 1200 µm, such as 100 to 1000 µm, such as 250 to 750 µm.

3. The rolled foil according to any one of the preceding claims, wherein:
a. the pharmaceutical composition is prone to degradation in the intestinal lumen,
b. the pharmaceutical composition is a low-permeability drug,
c. the pharmaceutical composition comprises a polypeptide, such as insulin, and/or
d. the pharmaceutical composition comprises a probiotic.

4. The rolled foil according to any one of the preceding claims, wherein the covering layer comprises
a. an enteric coating, such as an enteric coating that is inert below pH 5 and soluble above pH 5,
b. a gelatin capsule or a hydroxypropyl methylcellulose capsule, and/or
c. an enteric capsule or a pullulan capsule.

5. The rolled foil according to any one of the preceding claims, wherein the foil is made of:
a. a material that supports elastic deformation at strains of up to 50 %,
b. a material that retains a substantial amount of its elastic deformation over at period of 3 months,
c. a biorubber or an elastomer, such as a thermoplastic elastomer,
d. polydimethylsiloxane (PDMS), and/or
e. a material with a Young's Modulus of 0.1 to 100 MPa, such as 1 to 10 MPa.

6. The rolled foil according to any one of the preceding claims, wherein the radial pressure is 0.1 to 3000 Pa, such as 0.1 to 1 Pa, such as 1 to 5 Pa, such as 5 to 10 Pa, such as 10 to 20 Pa, such as 20 to 50 Pa, such as 50 to 200 Pa, such as 200 to 500 Pa, such as 500 to 1000 Pa, such as 1000 to 2000 Pa, such as 2000 to 3000 Pa.

7. The rolled foil according to any one of the preceding claims, wherein at least part of the surface of the foil is surface-treated to increase the hydrophilicity of the surface of the foil.

8. The rolled foil according to any one of the preceding claims, wherein the microstructures are ridges that define compartments, such as wherein the ridges are interconnected, such as wherein the ridges are 100 to 300 µm tall and/or wherein the ridges are 20 to 300 µm wide, such as wherein the ridge height/width aspect ratio is between 1 and 5.

9. The rolled foil according to claim 8, wherein the compartments are hexagonal.

10. The rolled foil according to claim 1, wherein the microstructures are needles.

11. The rolled foil according to any one of the preceding claims, wherein the pharmaceutical composition is situated on the foil material.

12. The rolled foil according to any one of claims 8 and 9, wherein the pharmaceutical composition is situated in the compartments.

13. The rolled foil according to any one of the preceding claims, wherein
a. the designated intestine is the small intestine and the length of the foil is at least 3.9 cm, such as between 6.0 and 10 cm, or
b. wherein the designated intestine is the large intestine and the length of the foil is at least 7.5 cm, such s between 12 and 18 cm.

14. The rolled foil according to any one of the preceding claims, wherein the convex surface of the rolled foil comprising the pharmaceutical composition is sealed with a sealing layer, such as:
a. wherein the sealing layer has a thickness of 10 to 50 µm,
b. wherein the sealing layer comprises a gel-sol transition promoter,
c. wherein the sealing layer comprises an extended release agent,
d. wherein the sealing layer comprises a delayed release agent,
e. wherein the sealing layer comprises an enteric coating, and/or
f. wherein the sealing layer is flexible

15. A method of manufacturing the rolled foil according to claim 1 comprising the steps of:
a. providing a foil that exerts a radial pressure when rolled to a diameter of the designated intestine wherein the foil has a length of at least half the circumference of the designated intestine,
b. loading the foil with the pharmaceutical composition, and optionally applying a sealing layer,
c. rolling the foil, and
d. covering the rolled foil in a covering layer.

## Patentansprüche

1. Gerollte Folie, die auf der konvexen Oberfläche der gerollten Folie eine pharmazeutischen Zusammensetzung aufweist, wobei die gerollte Folie und die pharmazeutische Zusammensetzung innerhalb einer Deckschicht umfasst sind, wobei die konvexe Oberfläche der gerollten Folie einen Radialdruck ausübt, wenn sie auf einen Durchmesser eines bezeichneten Darms abgerollt wird, wobei die konvexe Oberfläche Mikrostrukturen umfasst und wobei die Länge der gerollten Folie mindestens die Hälfte der Länge des inneren Umfangs des bezeichneten Darms beträgt.

2. Gerollte Folie nach Anspruch 1, wobei:
a. die gerollte Folie eine oder mehrere Windungen umfasst, wie z. B. mindestens zwei Windungen, wie z. B. mindestens drei Windungen,
b. die gerollte Folie einen Außendurchmesser von weniger als 9,55 mm aufweist, wie z. B. einen Außendurchmesser von weniger als 9,40 mm, wie z. B. einen Außendurchmesser von weniger als 8, 25 mm, wie z. B. einen Außendurchmesser von weniger als 8,00 mm,
c. die Breite der Folie weniger als 30 mm beträgt, wie z. B 8 bis 26 mm, und/oder
d. wobei die Folie eine Massendicke von 100 bis 1200 µm aufweist, wie z. B. 100 bis 1000 um, wie z. B. 250 bis 750 µm.

3. Gerollte Folie nach einem der vorhergehenden Ansprüche, wobei:
a. die pharmazeutische Zusammensetzung anfällig für eine Zersetzung in dem Darmlumen ist,
b. die pharmazeutische Zusammensetzung ein Arzneimittel mit geringer Permeabilität ist,
c. die pharmazeutische Zusammensetzung ein Polypeptid umfasst, wie z. B. Insulin und/oder
d. die pharmazeutische Zusammensetzung ein Probiotikum umfasst.

4. Gerollte Folie nach einem der vorhergehenden Ansprüche, wobei die Deckschicht
a. eine magensaftresistente Beschichtung, wie z. B. eine magensaftresistente Beschichtung, die unter pH 5 inert und über pH 5 löslich ist,
b. eine Gelatinekapsel oder eine Hydroxypropylmethylcellulosekapsel und/oder
c. eine magensaftresistente Kapsel oder eine Pullulankapsel umfasst.

5. Gerollte Folie nach einem der vorhergehenden Ansprüche, wobei die Folie hergestellt ist aus:
a. einem Material, das eine elastische Verformung bei Dehnungen von bis zu 50 % unterstützt,
b. einem Material, das eine erhebliche Menge der elastischen Verformung über einen Zeitraum von 3 Monaten beibehält,
c. einem Biokautschuk oder einem Elastomer, wie z. B. einem thermoplastischen Elastomer,
d. Polydimethylsiloxan (PDMS) und/oder
e. einem Material mit einem Youngscher-Modul von 0,1 bis 100 MPa, wie z. B. 1 bis 10 MPa.

6. Gerollte Folie nach einem der vorhergehenden Ansprüche, wobei der Radialdruck 0, 1 bis 3000 Pa, wie z. B. 0,1 bis 1 Pa, wie z. B. 1 bis 5 Pa, wie z. B. 5 bis 10 Pa, wie z. B. 10 bis 20 Pa, wie z. B. 20 bis 50 Pa, wie z. B. 50 bis 200 Pa, wie z. B. 200 bis 500 Pa, wie z. B. 500 bis 1000 Pa, wie z. B. 1000 bis 2000 Pa, wie z. B. 2000 bis 3000 Pa beträgt.

7. Gerollte Folie nach einem der vorhergehenden Ansprüche, wobei mindestens ein Teil der Oberfläche der Folie oberflächenbehandelt ist, um die Hydrophilie der Oberfläche der Folie zu erhöhen.

8. Gerollte Folie nach einem der vorhergehenden Ansprüche, wobei die Mikrostrukturen Rippen sind, die Fächer definieren, wie z. B. wobei die Rippen miteinander verbunden sind, wie z. B. wobei die Rippen 100 bis 300 µm hoch sind und/oder wobei die Rippen 20 bis 300 µm breit sind, wie z. B. wobei das Höhen/Breiten-Aspektverhältnis der Rippen zwischen 1 und 5 liegt.

9. Gerollte Folie nach Anspruch 8, wobei die Fächer sechseckig sind.

10. Gerollte Folie nach Anspruch 1, wobei die Mikrostrukturen Nadeln sind.

11. Gerollte Folie nach einem der vorhergehenden Ansprüche, wobei sich die pharmazeutische Zusammensetzung auf dem Folienmaterial befindet.

12. Gerollte Folie nach einem der Ansprüche 8 und 9, wobei sich die pharmazeutische Zusammensetzung in den Fächern befindet.

13. Gerollte Folie nach einem der vorhergehenden Ansprüche, wobei
a. der bezeichnete Darm der Dünndarm ist und die Länge der Folie mindestens 3,9 cm, wie z. B. zwischen 6,0 und 10 cm, beträgt oder
b. wobei der bezeichnete Darm der Dickdarm ist und die Länge der Folie mindestens 7,5 cm beträgt, wie z. B. zwischen 12 und 18 cm.

14. Gerollte Folie nach einem der vorhergehenden Ansprüche, wobei die konvexe Oberfläche der gerollten Folie, welche die pharmazeutische Zusammensetzung umfasst, mit einer Dichtungsschicht versiegelt ist, wie z. B.:
a. wobei die Dichtungsschicht eine Dicke von 10 bis 50 µm aufweist,
b. wobei die Dichtungsschicht einen Gel-Sol-Übergangspromotor umfasst,
c. wobei die Dichtungsschicht ein ausgedehntes Trennmittel umfasst,
d. wobei die Dichtungsschicht ein verzögertes Trennmittel umfasst,
e. wobei die Dichtungsschicht eine magensaftresistente Beschichtung umfasst und/oder
f. wobei die Dichtungsschicht flexibel ist.

15. Verfahren zur Herstellung der gerollten Folie nach Anspruch 1, umfassend die folgenden Schritte:
a. Bereitstellen einer Folie, die einen Radialdruck ausübt, wenn sie auf einen Durchmesser des bezeichneten Darms gerollt ist, wobei die Folie eine Länge von mindestens der Hälfte des Umfangs des bezeichneten Darms aufweist,
b. Beladen der Folie mit der pharmazeutischen Zusammensetzung und optionales Aufbringen einer Dichtungsschicht,
c. Aufrollen der Folie und
d. Bedecken der gerollten Folie mit einer Deckschicht.

## Revendications

1. Feuille enroulée ayant sur la surface convexe de la feuille enroulée une composition pharmaceutique, dans laquelle la feuille enroulée et la composition pharmaceutique sont comprises à l'intérieur d'une couche de recouvrement, dans laquelle la surface convexe de la feuille enroulée exerce une pression radiale lorsqu'elle est déroulée jusqu'à un diamètre d'un intestin désigné, dans laquelle la surface convexe comprend des microstructures, et dans laquelle la longueur de la feuille enroulée est au moins la moitié de la longueur de la circonférence interne de l'intestin désigné.

2. Feuille enroulée selon la revendication 1, dans laquelle :
a. la feuille enroulée comprend un ou plusieurs tours, par exemple au moins deux tours, par exemple au moins trois tours,
b. la feuille enroulée a un diamètre externe inférieur à 9,55 mm, par exemple un diamètre externe inférieur à 9,40 mm, par exemple un diamètre externe inférieur à 8,25 mm, par exemple un diamètre externe inférieur à 8,00 mm,
c. la largeur de la feuille est inférieure à 30 mm, par exemple de 8 à 26 mm, et/ou
d. dans laquelle la feuille a une épaisseur apparente de 100 à 1200 um, par exemple 100 à 1000 µm, par exemple 250 à 750 µm.

3. Feuille enroulée selon l'une quelconque des revendications précédentes, dans laquelle :
a. la composition pharmaceutique est sujette à la dégradation dans la lumière intestinale,
b. la composition pharmaceutique est un médicament à faible perméabilité,
c. la composition pharmaceutique comprend un polypeptide, tel que l'insuline, et/ou
d. la composition pharmaceutique comprend un probiotique.

4. Feuille enroulée selon l'une quelconque des revendications précédentes, dans laquelle la couche de recouvrement comprend
a. un enrobage entérique, par exemple un enrobage entérique qui est inerte en dessous de pH 5 et soluble au-dessus de pH 5,
b. une capsule de gélatine ou une capsule d'hydroxypropylméthylcellulose, et/ou
c. une capsule entérique ou une capsule de pullulane.

5. Feuille enroulée selon l'une quelconque des revendications précédentes, dans laquelle la feuille est constituée :
a. d'un matériau qui supporte une déformation élastique à des contraintes allant jusqu'à 50 %,
b. d'un matériau qui conserve une quantité substantielle de sa déformation élastique sur une période de 3 mois,
c. d'un bio-caoutchouc ou d'un élastomère, par exemple un élastomère thermoplastique,
d. de polydiméthylsiloxane (PDMS), et/ou
e. d'un matériau avec un module de Young de 0,1 à 100 MPa, par exemple de 1 à 10 MPa.

6. Feuille enroulée selon l'une quelconque des revendications précédentes, dans laquelle la pression radiale est de 0,1 à 3000 Pa, par exemple de 0,1 à 1 Pa, par exemple de 1 à 5 Pa, par exemple de 5 à 10 Pa, par exemple de 10 à 20 Pa, par exemple de 20 à 50 Pa, par exemple de 50 à 200 Pa, par exemple de 200 à 500 Pa, par exemple de 500 à 1000 Pa, par exemple de 1000 à 2000 Pa, par exemple de 2000 à 3000 Pa.

7. Feuille enroulée selon l'une quelconque des revendications précédentes, dans laquelle au moins une partie de la surface de la feuille est traitée en surface pour augmenter l'hydrophilie de la surface de la feuille.

8. Feuille enroulée selon l'une quelconque des revendications précédentes, dans laquelle les microstructures sont des crêtes qui définissent des compartiments, par exemple dans laquelle les crêtes sont interconnectées, par exemple dans laquelle les crêtes ont une hauteur de 100 à 300 µm et/ou dans laquelle les crêtes ont une largeur de 20 à 300 um, par exemple dans laquelle le rapport d'aspect hauteur/largeur de crête est compris entre 1 et 5.

9. Feuille enroulée selon la revendication 8, dans laquelle les compartiments sont hexagonaux.

10. Feuille enroulée selon la revendication 1, dans laquelle les microstructures sont des aiguilles.

11. Feuille enroulée selon l'une quelconque des revendications précédentes, dans laquelle la composition pharmaceutique est située sur le matériau en feuille.

12. Feuille enroulée selon l'une quelconque des revendications 8 et 9, dans laquelle la composition pharmaceutique est située dans les compartiments.

13. Feuille enroulée selon l'une quelconque des revendications précédentes, dans laquelle
a. l'intestin désigné est l'intestin grêle et la longueur de la feuille est d'au moins 3,9 cm, par exemple entre 6,0 et 10 cm, ou
b. dans laquelle l'intestin désigné est le gros intestin et la longueur de la feuille est d'au moins 7,5 cm, par exemple entre 12 et 18 cm.

14. Feuille enroulée selon l'une quelconque des revendications précédentes, dans laquelle la surface convexe de la feuille enroulée comprenant la composition pharmaceutique est scellée avec une couche de scellement, par exemple :
a. dans laquelle la couche de scellement a une épaisseur de 10 à 50 pm,
b. dans laquelle la couche de scellement comprend un promoteur de transition gel-sol,
c. dans laquelle la couche de scellement comprend un agent de libération prolongée,
d. dans laquelle la couche de scellement comprend un agent de libération retardée,
e. dans laquelle la couche de scellement comprend un enrobage entérique, et/ou
f. dans laquelle la couche de scellement est flexible.

15. Procédé de fabrication de la feuille enroulée selon la revendication 1, comprenant les étapes suivantes :
a. la fourniture d'une feuille qui exerce une pression radiale lorsqu'elle est enroulée jusqu'à un diamètre de l'intestin désigné, dans lequel la feuille a une longueur d'au moins la moitié de la circonférence de l'intestin désigné,
b. le chargement de la feuille avec la composition pharmaceutique, et éventuellement l'application d'une couche de scellement,
c. l'enroulement de la feuille, et
d. le recouvrement de la feuille enroulée d'une couche de recouvrement.
